# EUROPÄISCHE PATENTSCHRIFT

(11) **EP 1 327 450 B1**
(45) Veröffentlichungstag und Bekanntmachung des Hinweises auf die Patenterteilung: **15.08.2007**
(21) Anmeldenummer: 02000901.5
(22) Anmeldetag: 15.01.2002
(51) Int. Cl.: A61K 39/00, A61K 39/39

(54) **Orale Vakzinierung mit nackten Tumor-Antigen-Mimotopen**
Oral vaccination with naked tumor-antigen-mimotopes
Vaccination orale par des mimotopes d'antigene tumoraux dénudés

(43) Veröffentlichungstag der Anmeldung: 16.07.2003
(73) Patentinhaber: Bio Life Science Forschungs- und Entwicklungsges.m.b.H., 1010 Wien (AT)
(72) Erfinder: Jensen-Jarolim, Erika, A-1210 Wien (AT); Scheiner, Otto, A-2380 Perchtoldsdorf (AT); Pehamberger, Hubert, A-1230 Wien (AT); Zielinski, Christoph, A-1180 Wien (AT); Breiteneder, Heimo, A-1070 Wien (AT)
(74) Vertreter: Kador & Partner

(56) Entgegenhaltungen:
- WO-A-96/33732
- WO-A-98/43479
- WO-A1-01/08495
- WO-A1-99/07869
- US-A- 4 171 353
- US-A- 5 840 332
- DATABASE [Online] 2005-10-24 14:23:21.0 'Gastric Acid Inhibitors' Retrieved from HTTP://WWW.SURGERYENCYCLOPEDIA.COM/FI-LA/GA STRIC-ACID-INHIBITORS.HTML
- LAHEIJ R.J. ET AL: 'Medical biology pneumonia and gastric-acid suppressive drugs' J AM MED ASSOC, [Online] Bd. 292, 2004, Seite 1955 Gefunden im Internet: <URL:http://scienceweek.com/2004/sc041217-2 .htm>
- Hochschule für angewandte Wissenschaften Hamburg, Fakultät Life Science, Studiengang Ökotrophologie, Anatomie/Physiologie, Klausur im SS2005 am 29.6.2005, Prof. Dr. Lorenz
- GENGLBERGER E. ET AL: 'monovalent fusion proteins if IgE mimotopes are safe for therapy of type I allergy' THE FASEB JOURNAL Bd. 15, November 2001, Seiten 2524 - 2526
- ZUERCHER A.W. ET AL: 'Oral anti-IgE immunization with epitope-displaying phage' EUROPEAN JOURNAL OF IMMUNOLOGY Bd. 30, 2000, Seiten 128 - 135

## Beschreibung

Die vorliegende Erfindung betrifft die verwendung eines Kombinationspräparates als ein Arzneimittel zur oralen Vakzinierung.

In der Medizin ist es häufig erwünscht, dass Krankheiten nicht nur symptomatisch bekämpft werden, sondern ihr Entstehen von vorne herein verhindert wird. Im Bereich der Infektionskrankheiten, neuerdings aber auch im Bereich der Krebserkrankungen wird daher der Weg der Vakzinierung eingeschlagen. Mittels dieser Methode wird der Körper dazu befähigt, mit Hilfe seines Immunsystems auf krankheitserregende Substanzen, auch Antigene genannt, zu reagieren. Dieses auch als aktive Immunisierung bekannte Verfahren funktioniert dadurch, dass dem Immunsystem ein mit einer Krankheit assoziiertes Antigen präsentiert wird, worauf das Immunsystem mit der Bildung entsprechender spezifischer Antikörper gegen besagte Antigene reagiert. Diese sogenannten Antigene bestehen häufig aus Peptiden oder Proteinen, dass heißt Aminosäuresequenzen, welche häufig parenteral, das heißt unter Umgehung des Magen-Darm-Traktes verabreicht werden. Dies geschieht meist mittels Injektion oder Infusion, was von den Patienten häufig als unangenehm empfunden wird.

Dem gemäß ist es Aufgabe der vorliegenden Erfindung, eine Tumor-Vakzine zur Verfügung zu stellen, mit Hilfe derer es möglich ist, eine Impfung auf parenteralem Wege zu vermeiden und die Akzeptanz bei den Patienten dadurch zu steigern.

US4171353 beschreibt die Verwendung von Cholinen als Vakzin-Adjuvans. Weiters beschreibt US4171353, dass bei oraler Immunisierung mit einem Antigen, die Gefahr besteht, dass dieses durch Magensäure angegriffen wird. Deshalb sei es wünschenswert Antigene gegen Magensäure zu schützen bis sie den Dünndarm erreichen. Drei veschiedene Möglichkeiten werden beschrieben: a) enterisches Ummanteln oder Verkapseln des Antigens; b) Formulierung des Antigens mit einer Magensäure- und Pepsin hemmenden/-blockierenden Substanz, wie z.B. Antihistaminika wie Cimetidin (= H2-Rezeptorblocker); c) Formulierung des Antigens mit einem alkalischen Material/Puffer wie z.B. Na-Bikarbonat (=Antazid) um die Magensäure zu neutralisieren bzw. zu reduzieren. Als Antigen wirksame Subtanzen werden in US4171353 u.a. Viren oder Tumorzellen genannt.

ZUERCHER-AW et al. (Euro. J. Immunol. 30:128-135; 2000) beschreibt die erfolgreiche Induktion anti-IgE spezifischer Antikörper nach oraler Immunisierung mit mittels anti-IgE Antikörpern hergestellter Mimotope.

Der Erfindung liegt die Erkenntnis zugrunde, dass eine parenterale Applikation dann umgangen werden kann, wenn im Magenbereich geeignete Bedingungen herrschen, die die Wirksamkeit des Impfstoffes nicht beeinträchtigen.

Gegenstand der vorliegenden Erfindung ist die Verwendung gemäß Anspruch 1.

Durch diese Verwendung wird zum einen ermöglicht, ' dass die als Antigen wirksame Substanz gleichzeitig mit der magensäure-reduzierenden Substanz eingenommen werden kann, wobei beide Phasen zusammen oder getrennt vorliegen. Zum anderen kann das Kombinationspräparat auch in der Weise appliziert werden, dass die als Antigen wirksame Substanz getrennt und nach der Applikation einer geeigneten Dosis an magensäurereduzierender Substanz verabreicht wird. Dabei ist es wichtig, dass die Magensäure zum Zeitpunkt der Applikation, das heißt zu dem Zeitpunkt, an dem der Impfstoff in den Magen gelangt, im Vergleich zum "normalen Magensäuregehalt" reduziert ist. Auf diese Weise wird es ermöglicht, dass eine traditionell parenteral zu verabreichende Vakzine auch oral appliziert werden kann und ihre Wirkung in Form der Erzeugung einer Immunantwort entfalten kann. Des weiteren hat die reduzierte Azidität auch eine Nicht-Aktivierung der Magen-Proteasen zur Folge, denn Pepsinogene werden erst durch den niedrigen pH im Magen zu den aktiven Pepsinen gespalten.

Bevorzugter Weise dienen die Arzneimittel zur Vakzinierung von Säugetieren. Da auch Tiere sowohl Tumoren entwickeln können, als auch in der Lage sind, IgE zu produzieren, ist auch im veterinärmedizinischen Bereich an eine Vakzinierung gemäß der vorliegenden Erfindung zu denken. Besonders bevorzugt dienen die erfindungsgemäß verwendeten Arnzeimittel zur Vakzinierung von Menschen.

Bei einer gleichzeitigen Gabe von Arzneimitteln, die aus einer als Antigen wirksamen Substanz und einer magensäurereduzierenden Substanz bestehen, kann die als Antigen wirksame Substanz im Magen gleichzeitig oder mit zeitlicher Verzögerung im Vergleich zur magensäurereduzierenden Substanz abgegeben werden. Wichtig ist dabei nur, dass das Magenmilieu in der Weise eingestellt ist, dass die als Antigen wirksame Substanz ihre Wirkung bezüglich der Erzeugung einer Immunantwort entfalten kann. Pathophysiologisch oder iatrogen induziert können manche Individuen aus unterschiedlichen Gründen eine reduzierte Magensäure haben. Für die erfindungsgemäße Vakzinierung ist es jedoch notwendig, gezielt und kontrolliert den Säuregehalt zu beeinflussen.

Der genaue Wirkmechanismus ist noch unbekannt, es wird jedoch vermutet, dass das Antigen unter hypoaziden Bedingungen konformationell intakt bleibt, wobei Konformationsepitope möglicherweise für die Induktion von IgE Antikörpern ausschlaggebend sind. Eine erhöhte Resorptionsrate des Antigens durch die Magenwand konnte bisher noch nicht festgestellt werden.

Bevorzugt besteht die magensäurereduzierende Substanz aus einer die Magensäurebildung inhibierenden und/oder die Magensäure bindenden Substanz. Damit wird gewährleistet, dass die Art und Weise wie es zur Magensäurereduktion kommt auf unterschiedlichen Wirkmechanismen beruhen kann. Durch solche Mittel kann die Magensäurekonzentration auf die Bedürfnisse der erfindungsgemäßen Vakzinierung eingestellt werden.

Bevorzugt werden als magensäurereduzierende Mittel solche ausgewählt, die den Wirkstofffamilien der Antazida, H₂-Rezeptor-Antagonisten oder Protonenpumpeninhibitoren angehören. Durch das Verabreichen von Antazida, d.h. basischen Mitteln, wird vorhandene Magensäure reduziert. H₂-Rezeptor-Antagonisten inhibieren hingegen Histamin H₂-Rezeptoren kompetitiv und reversiv, wobei die Magensäurebildung vermindert wird. Protonenpumpeninhibitoren reduzieren ihrerseits die Magensäure durch direkte Beeinflussung der Säuresekretion.

Als Beispiele für Antazida seien folgende Wirkstoffe genannt: Natriumhydrogencarbonat, Calciumcarbonat, Magnesiumcarbonat, Magnesiumhydroxid und Magnesiumhydroxid-Gel, Magnesiumsilikat, Aluminiumphosphat, Aluminiumhydroxid und Aluminiumhydroxid-Gel, Hydrotalcite, Magaldrate, Dihydro-Aluminium-Natriumcarbonat, Magnesium-Aluminathydrat, Aminoessigsäure sowie Wismuth-Salze. Als protektiv über die Schleimhaut wirksame Substanzen seien Carbenoxolon und Sucralat (Aluminiumhydroxid und Saccharosesulfat) zu nennen. Als Beispiele für H₂-Rezeptorblocker seien exemplarisch genannt: Cimetidin, Ranitidin, Oxmetidin, Famotidin, Roxatidin und Nizatidin. Als Beispiele für Protonenpumpeninhibitoren seien genannt: Omezaprol, Lansoprazol, Pantoprazol und Rabeprazol. Da Anticholinergika u.a. eine Hemmung der Speichel- u. Magensaftabsonderung bewirken, können auch Vertreter dieser Klasse verwandt werden, so z.B. Pirenzepin.

Besonders bevorzugt werden solche magensäurereduzierenden Mittel aus den Mitteln Ranitidin-Hydrochlorid und Aluminium-Saccharose-HydrogenSulfat ausgewählt. Beide Mittel sind für die erfindungsgemäßen Arzneimittel geeignet und im allgemeinen gut verträglich.

Als Antigen wirksame Substanzen werden vorzugsweise natürliche oder synthetische Tumor-Antigen-Mimotope verwendet. Die verwendeten natürlichen oder synthetischen Tumor-Antigen-Mimotope oder Gemische davon bewirken nach der oralen Einnahme eine Immunantwort, die sich auf die Bildung von Immunglobulinen bezieht. Bevorzugt werden die Immunglobuline IgE und IgG1 gebildet.

Es ist weiterhin möglich, dass die erfindungsgemäß verwendeten Arzneimittel Tumor- Antigen-Mimotope als Monomere, Dimere, Trimere oder Oligomere enthalten. Hier wird eine erhöhte Immunogenität erzeugt.

In Bezug auf die Einsetzbarkeit der als Antigen fungierenden Substanzen in den erfindungsgemäß verwendeten Arzneimitteln sind zahlreiche Möglichkeiten denkbar.

Die erfindungsgemäß verwendeten Arzneimittel, besitzen die als Antigen wirksame Substanz die eine antitumorale Wirkung auslöst. Gerade im Bereich der Krebstherapie und Krebsvorsorge sind Vakzinierungen und insbesondere der Vorteil einer möglichen oralen Vakzinierung für die Patienten erwünscht.

Nachstehend sind Beispiele einer oralen Vakzinierung erläutert, die zum Verständnis der Erfindung beitragen. Zur Erklärung zeigt:

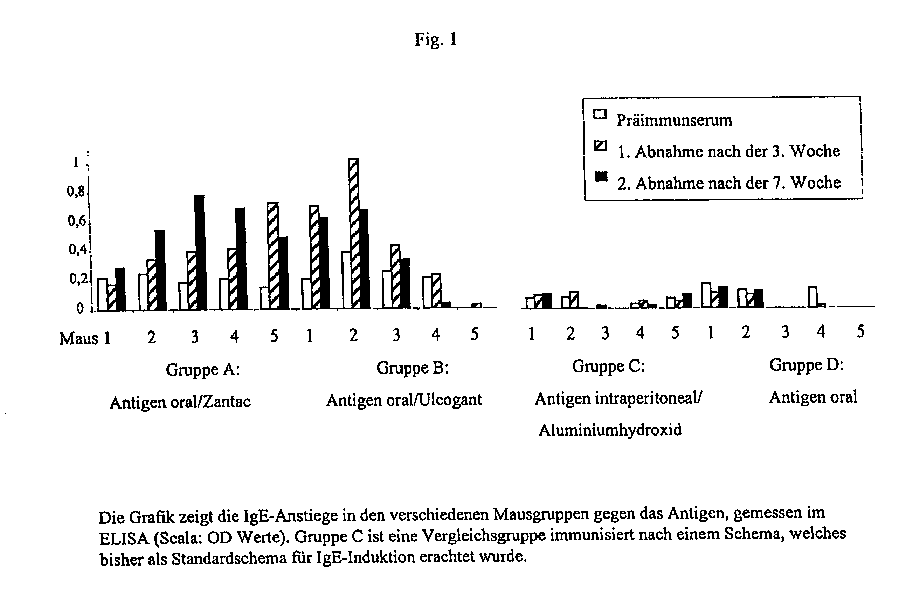

Figur 1: Den IgE-Anstieg in den verschiedenen Mausgruppen gegen das Antigen, gemessen im ELISA (Scala: OD-Werte).
   **Gruppe A**: Orale Gabe von Zantac und Antigen
   **Gruppe B**: Orale Gabe von Ulcogant und Antigen
   **Gruppe C**: Vergleichsbeispiel mit intraperitonealer Gabe von Antigen und Aluminiumhydroxid als Adjuvans
   **Gruppe D**: Orale Gabe des Antigens

Befunde gemäß der vorliegenden Erfindung machen deutlich, dass eine Hypoazidität des Magens (durch Antazida-Behandlung z.B. wegen eines Ulcus venticuli) bei gleichzeitiger erstmaligen Einnahme eines Antigens zu einer IgE Induktion spezifisch gegen dieses neue Antigen führt. Diese Situation wurde im Tiermodell überprüft und folgende Resultate wurden erhalten (Figur 1):

Gängige Präparate, welche die Magensäurebildung inhibieren (Zantac^{®} [Ranitidin-Hydrochlorid, GlaxoSmithKline]) oder welche die Magensäure binden und neutralisieren (Ulcogant^{®} [Aluminium-Saccharose-Hydrogensulfat, Merck]), bewirken eine nachhaltige Induktion von IgE und auch IgG Antikörpern gegen ein gleichzeitig gefüttertes neues Nahrungsmittel, jedoch nicht gegen die tägliche Diät der Mäuse, gegen welche sie weiter tolerant blieben. Die erzielten IgE-Werte sind auch im Vergleich zu einer traditionellen Allergisierung auf intraperitonealem Weg mit Al(OH)₃ als Adjuvans hoch. Aufgrund der vermehrten Literaturberichte, welche auf eine anti-Tumorwirkung von IgE Antikörpern hinweisen (Reali, E.; Greiner, J.W.; Corti, A.; Gould, H.J., Bottazzoli, F.; Paganelli, G.; Schlom, J.; Siccardi, A.G.; Cancer Res., 2001; 61(14): 5517-22; Kershaw, M.H., Darcy, P.K.; Trapani, J.A.; MacGregor, D.; Smyth, m.J.; Oncol. Res., 1998, 10(3), 133-42; Neuchrist, C.; Kornfehl, J.; Grasl, M.; Lassmann, H.; Kraft, D.; Ehrenberger, K.; Scheiner, O.; Int. Archs. Allergy Immunol., 1994, 104: 97-100; Nagy, E.; Berczi, I.; Sehon, A.H.; Cancer Immunol. Immunother. 1991; 34(1): 63-9), kann mit einem positiven Effekt einer Vakzinierung durch orale Applikation von Tumor-Antigen-Mimotope gerechnet werden.

## Patentansprüche

1. Verwendung eines Kombinationspräparats, bestehend aus einer als Antigen wirksamen Substanz und einer magensäurereduzierenden Substanz, zur Herstellung einer Vakzine gegen Tumorerkrankungen, wobei die Antigen wirksame Substanz ein Tumor Antigenmimotop ist und die magensäurereduzierende Substanz gemeinsam oder getrennt, gleichzeitig öder zeitlich versetzt, oral angewendet werden, und die als Antigen wirksome substanz eine anti-tumorale Wirkung auslöst.

2. Verwendung nach Anspruch 1, **dadurch gekennzeichnet, dass** die als Antigen wirksame Substanz im Magen gleichzeitig oder mit zeitlicher Verzögerung im Vergleich zur magensäurereduzierenden Substanz freigesetzt wird.

3. Verwendung nach Anspruch 1 oder 2, **dadurch gekennzeichnet, dass** die magensäurereduzierende Substanz die Magensäurebildung inhibiert und/oder Magensäure bindet.

4. Verwendung nach Anspruch 3, **dadurch gekennzeichnet, dass** die magensäurereduzierende Substanz aus Mitteln der Wirkstofffamilien der Antazida, H2-Rezeptor-Antagonisten und/oder Protonenpumpeninhibitoren ausgewählt wird.

## Claims

1. Use of a combination preparation comprising an antigenically active substance and a gastric acid reducing substance for producing a vaccine for tumoral diseases, wherein the antigenically active substance is a tumor antigen mimotope and is applied orally with the gastric acid reducing substance jointly or separately, simultaneously or staggered, and the antigenically active substance triggers an antitumoral effect.

2. The use according to claim 1, **characterized in that** the antigenically active substance is released in the stomach simultaneously or with a time delay in comparison with the gastric acid reducing substance.

3. The use according to claim 1 or 2, **characterized in that** the gastric acid reducing substance inhibits gastric acid formation and/or binds gastric acid.

4. The use according to claim 3, **characterized in that** the gastric acid reducing substance is selected from agents of the families of active agents of antacids, H₂- receptor antagonists and/or proton pump inhibitors.

## Revendications

1. Utilisation d'une préparation combinée, constituée d'une substance active en tant qu'antigène et d'une substance réduisant la quantité d'acide gastrique, pour la fabrication d'un vaccin contre des maladies tumorales, la substance active en tant qu'antigène consistant en un mimotope d'antigène tumoral, et la substance active en tant qu'antigène et la substance réduisant la quantité d'acide gastrique étant administrées par voie orale, conjointement ou séparément, simultanément ou à un certain intervalle de temps, et la substance active en tant qu'antigène déclenchant un effet antitumoral.

2. Utilisation selon la revendication 1, **caractérisée en ce que** la substance active en tant qu'antigène est libérée dans l'estomac en même temps que ou avec un certain retard par rapport à la substance réduisant la quantité d'acide gastrique.

3. Utilisation selon la revendication 1 ou 2, **caractérisée en ce que** la substance réduisant la quantité d'acide gastrique inhibe la formation d'acide gastrique et/ou fixe l'acide gastrique.

4. Utilisation selon la revendication 3, **caractérisée en ce que** la substance réduisant la quantité d'acide gastrique est choisie parmi des agents appartenant aux familles de principes actifs des antiacides, des antagonistes de récepteurs H₂ et/ou des inhibiteurs de pompe à protons.
